Europäisches Patentamt

European Patent Office

Office européen des brevets

(11) Publication number: **0 239 335**
**A2**

# EUROPEAN PATENT APPLICATION

(21) Application number: 87302432.7

(22) Date of filing: 20.03.87

(51) Int. Cl.4: **C08B 37/08 , A61L 31/00**

(30) Priority: 21.03.86 US 842634

(43) Date of publication of application:
30.09.87 Bulletin 87/40

(84) Designated Contracting States:
AT BE CH DE ES FR GB IT LI NL SE

(71) Applicant: INTERNATIONAL
PHARMACEUTICAL PRODUCTS, INC.
3001 Red Hill Avenue Suite 222 Esplanade IV
Costa Mesa California 92626(US)

(72) Inventor: Hildesheim, Jean c/o International
Pharmaceutical
Products, Inc. 3001 Red Hill Avenue Suite
222
Esplanade IV§Costa Mesa§California
92626(US)

(74) Representative: Myerscough, Philip Boyd et al
J.A.Kemp & Co. 14, South Square Gray's Inn
London, WC1R 5EU(GB)

(54) Non-inflammatory hyaluronic acid fraction and process for preparing it.

(57) A sterile, non-inflammatory, heat-resistant hyaluronic acid fraction of low molecular weight, and a method for extracting it from a crude source. The hyaluronic acid fraction is obtained by washing a deproteinized crude source with butanol, precipitating a hyaluronic acid-quaternaryalkylamine complex, dissolving the complex in an excess of sodium dodecylsulfate solution, and precipitating the sterile, non-inflammatory, heat-resistant hyaluronic acid fraction from the soution.

EP 0 239 335 A2

## NON-INFLAMMATORY HYALURONIC ACID FRACTION AND PROCESS FOR PREPARING IT

FIELD OF INVENTION

This invention relates generally to hyaluronic acid and a process for obtaining a sterile, non-inflammatory, heat resistant fraction thereof.

BACKGROUND OF THE INVENTION

Hyaluronic acid (hereinafter referred to as HA) is a naturally occurring compound found in a variety of animal tissues including, for example, vitreous humor, synovial fluid, umbilical cord, rooster combs, and pathologic joints. HA is also found in group A and C hemolytic streptococci. The compound is a highly viscous mucopolysaccharide with alternating $\beta$ 1-3 glucuronic and $\beta$ 1-4 glucosaminidic bonds, and it has a molecular weight ranging from 50,000 to 8 x $10^6$ daltons. The isolation and characterization of HA have been well described in Meyer, et al., J. Biol. Chem. 107, 629 (1934); J. Biol. Chem. 114, 689 (1936); Balazs, Fed. Proc. 17, 1086 (1958); Laurent, et al., Biochem. Biophys. Acta. 42, 476 (1960); Weissman, et al., J. Am. Chem. Soc. 76, 1753 (1954); and Meyer, Fed. Proc. 17, 1075 (1958).

At the present time, HA is frequently used in ocular surgery as a replacement for subretinal fluid and vitreous humor that are lost during surgery. HA has other therapeutic uses, including use as a post-operative preparation for the prevention of tissue adhesion and scarring. HA can also be used as a replacement for synovial fluid that is lost as a result of surgery or chronic inflammatory disease such as rheumatoid arthritis.

The use of HA as a therapeutic agent, however, has been dramatically limited due to the inability of researchers to obtain non-inflammatory extracts from animal tissue. Nearly all techniques for the isolation of HA produce an HA with an unknown contaminant that stimulates an inflammatory response. Thus, although HA is a useful replacement for the body's fluids, such as vitreous humor or synovial fluid, clinicians have either chosen not to use HA or have used HA in combination with non-inflammatory drugs that may produce other undesirable side-effects.

A method for the preparation of a non-inflammatory HA has been described in U.S. Patent No. 4,141,973 to Balazs (incorporated herein by reference and hereinafter referred to as Balazs). Balazs uses chloroform to prepare a high molecular weight HA fraction with a molecular weight greater than 750,000 that is said to be non-inflammatory. The Balazs method, however, is costly and low yielding. Chloroform is also highly photosensitive and upon exposure to light will react with a wide variety of chemical entities that could end up as impurities in the final HA preparation. Moreover, a chloroform is highly toxic and its use in the preparation of pharmaceuticals is less than desirable. The high molecular weight HA derived from the Balazs procedure is also heat sensitive and may degrade upon exposure to high temperature. Therefore, the Balazs HA fraction is sterilized by a chemical process rather than heat. Since most therapeutic applications of HA require sterilization, the chemical approach of Balazs further lengthens the time and expense of preparing HA.

It is an object of the present invention to provide a novel high yield procedure for the extraction of non-inflammatory HA.

It is further an object of the present invention to provide a low molecular weight non-inflammatory HA which can be heated and then cooled without losing its physical or chemical properties.

SUMMARY OF THE INVENTION

The present invention provides a sterile, non-inflammatory, heat resistant HA fraction of low molecular weight, and a method for extracting it from a crude source. According to the present invention, deproteinized and nucleic acid poor HA is washed with butanol and precipitated in an excess of a quarternaryalkyl or aryl amine (hereinafter referred to as a QAA) such as cetyl pyridinum chloride (hereinafter referred to as CPC) or hexadecyltrimethyl ammonium bromide (hereinafter referred to as HTA). The precipitated QAA-HA complex is thereafter redissolved with an excess of sodium dodecylsulfate (hereinafter referred to as SDS) and the HA precipitated by ethanol. This procedure yields a sterile, non-inflammatory, heat resistant HA of low molecular weight that can be used in the same manner as previous HA extractions without the need for non-inflammatory drugs.

An added advantage of the HA fraction of the present invention is its ability to withstand heating to high temperatures and subsequent cooling without a loss of its physical or chemical properties. At physiological temperatures (approximately 37 degrees C) the HA fraction of the present invention is highly viscous. As the HA fraction is heated to high temperatures, viscosity is lost. As the HA fraction is subsequently cooled, viscosity returns to its previous level. The ability of the HA fraction of the present invention to withstand high temperatures provides the added advantage that the HA fraction can be sterilized by heat rather than by a chemical process. Further, because viscosity is lost in high temperatures, filtration of the HA fraction of the present invention as high temperatures for sterilization or further purification is easier.

In the more detailed aspects of the invention, the step of deproteinizing HA may be preceded by an initial step of treating the crude HA with ethanol. The ethanol-treated HA is then treated with a mixture of DNase and RNase for 16 hours to digest any nucleic acids present. The HA is then deproteinized with a 1% protease solution. The mixture is incubated until the protease reaction is complete, approximately four hours.

The step of washing the deproteinized HA with butanol follows the protease treatment. Butanol is stirred into the HA along with an excess of a nonorganic ionic salt such as NaCl to wash the remaining protein from the HA fraction. The HA-containing aqueous phase is allowed to separate at 18-25 degrees C for approximately 15 to 20 hours. After filtration the aqueous phase is treated with ethanol.

The step of precipitating the HA with a QAA follows the butanol extraction. Various QAAS can be used; however, CPC or HTA is preferred. The extracted HA is resuspended in water and an excess of the QAA is added forming an insoluble QAA-HA complex. The precipitate QAA-HA complex is washed with water. An excess of SDS solution is added, which breaks down the QAA-HA complex, and the suspension stirred until the precipitate is dissolved. In the final step, the HA is ethanol precipitated, filtered, dried and redissolved in a physiologically acceptable saline buffer to the desired concentration.

Other aspects and advantages of the invention should become apparent from the following description of the preferred embodiment, which illustrates, by way of example, the principles of the invention.

DESCRIPTION OF THE PREFERRED EMBODIMENT

The present invention is embodied in a sterile, non-inflammatory, heat resistant hyaluronic acid fraction and a process for extracting it from a crude source. HA has had limited therapeutic use due to the inability of researchers to obtain non-inflammatory extracts from crude source such as animal tissue. Although HA may be useful as a replacement for the body's natural fluids such as vitreous humor or synovial fluid that are lost as a result of surgery or disease, clinicians have chosen not to use HA or have used HA in combination with non-inflammatory drugs that may produce other non-desirable side effects. Although a non-inflammatory HA can be obtained by the Balazs procedure, the procedure is costly and is low yielding. Moreover, the high molecular weight HA derived from the Balazs procedure is sterilized by a chemical process, which further lengthens the time and expense of the procedure.

The procedure of the present invention provides a sterile, non-inflammatory, heat resistant HA fraction of low molecular weight (less than 750,000). This HA fraction can be used in the same manner as previous HA extractions without the need of non-inflammatory drugs. The HA fraction has the added advantage that it can be conveniently sterilized and filtered at high temperatures. The HA fraction is obtained by washing deproteinized HA from a crude source with butanol and precipitating it in an excess of QAA. The precipitated QAA-HA complex is then dissolved in an excess of SDS and, after filtration, the now sterile, pyrogen-free, protein-free, non-inflammatory HA is precipitated with ethanol, dried and redissolved in a pharmaceutically acceptable carrier solution.

In the preferred embodiment of the present invention, eight grams of the crude HA is sterilized by suspending the HA in 125 ml of 95% pharmaceutical grade ethanol and mixed on a shaker at one or two strokes per second at room temperature (18-25) degrees C) for one hour. The suspension is then filtered through a 1 to 8 micron filter and rinsed with 15 ml portions of ethanol until all the hyaluronic acid is retained on the filter.

The HA is then dissolved in 1.3 liters of a sterile physiological sodium phosphate buffer pH 7.2 containing 12 ml of 0.4 M magnesium sulfate, and heated with stirring at 35-38 degrees C. DNase 50 $\mu$g and RNase 30 $\mu$g are added and the solution is incubated for 16 hours. Forty mg of protease in a 1% solution is then added and the solution mixed on a shaker at one or two strokes per second for four hours at 35-38 degrees C. Any protease which is effective at a neutral pH and devoid of Hyaluronidase activity, such as Streptomyces griseus protease (protease E.), may be used. The protease treatment is repeated twice, with the last treatment lasting 16 hours and monitored at 600 mn, to ensure complete digestion of all proteins. The resulting protein concentration as determined by the Bradford method is typically 0.2-0.3% of the total weight of HA and as the term is used herein is "protein-free."

After the protease treatment is completed, 650 ml of butanol is added and the solution mixed on a shaker at one or two strokes per second for 16 hours at 20-40 degrees C. An excess of 4M NaCl is then added and the solution is mixed for an additional 15 minutes.

The solution is allowed to stand for 15-20 hours at 18-25 degrees C for adequate phase separation. Thereafter the aqueous phase is drained and filtered through a 1-8 nm glass fiber filter, and 2.6 liters of ethanol are added. The solution is mixed slowly on the shaker for two hours. The supernant is discarded and 1.3 liters of water are added until the HA precipitate is completely dissolved. An excess of HTA solution, 240 ml, is then added to precipitate the HA, and the contents stirred gently for one hour. Other QAAs, such as CPC, can also be used. The supernant is discarded and the precipitate washed with 600 ml of water. The supernant is again discarded. An excess SDS solution in saline buffer, 1.3 liters, is added to the precipitate and mixed until the precipitate is completely dissolved. The solution is then filtered through a 1-2.2 nm filter and 2.6 liters of ethanol are added and the solution mixed slowly on the shaker for two hours at one or two strokes per second. The supernant is filtered through a sintered glass filter tube through aspiration and the HA precipitate is saved.

The HA precipitate is redissolved in 1.3 liters of saline buffer by stirring. The solution is filtered and the HA content determined by Carbazole reaction. The HA is precipitated by the addition of 1-2.6 liters of ethanol and mixed slowly on the shaker for two hours. The HA precipitate is dissolved in 1.3 liters saline. The solution is mixed slowly on the shaker for two hours at one or two strokes per second, filtered, and the supernant discarded. The HA precipitate is dried by aspiration under at least a 20 mm Hg vacuum for approximately four hours and resuspended in PBS or saline to a final HA concentration of between about 1.2 and 2.0 percent by weight.

The above procedure yields a highly pure pyrogen-free, nucleic acid-free, protein-free, non-inflammatory, heat resistant H fraction with an average molecular weight of less than 750,000. Although precipitating the HA of the present invention with a QAA also sterilizes the HA fraction, sterile procedures should be employed throughout the process.

A solution of the HA fraction of the present invention has the additional advantage of being capable of being heated to above 60 degrees C and less than 95 degrees C for several hours or days and subsequently cooled without a significant loss of its physical or chemical properties and, as the term is used herein, is "heat resistant." At these temperatures the HA becomes significantly less viscous and therefore easier to handle. The less viscous HA solution is particularly easy to pour, making it easier to measure the desired volume of HA solution. The less viscous HA solution can also be filtered to further sterilize or process the HA fraction. Accordingly, the HA fraction of the present invention can be easily prefilled into applicators such as surgical syringes.

The non-inflammatory properties of the HA fraction of the present invention were tested by use of a vitreous eye test on rabbits and cynomolgus monkeys. Both rabbits and cynomolgus monkeys were chosen because of the well known sensitivity of their eyes to inflammation. The vitreous eye test involves replacing aqueous humor from the animal's eye with the test solution. The animal is then monitored periodically for reddening flare around the eye or the migration of inflammatory cells into the vitreous. The results of the tests show that the HA fraction of the present invention does not initiate an inflammatory response.

Example I: Vitreous Test on Rabbits

Six healthy, adult rabbits with no known eye defects were selected in a random fashion to control bias. All animals were examined and those animals having grossly detectable eye defects as determined by biomicroscopic examination were eliminated. Each animal was restrained by an intramuscular injection of a mixture of Ketamine HCL, Xylazine, Acepromazine. (50 mg/kg, 12 mg/kg, and 1 mg/kg, respectively.) Additionally, two drops of Pontocaine HCL, 0.5 percent solution, topical anaesthetic, or the equivalent, was added to each eye before any manipulation occurred. After restraint, the animal's eye was examined for flair and cell response.

An amount of aqueous humor was then removed from the anterior chamber to moderately collapse the chamber. This was accomplished by inserting a 26-gauge needle with a 1.0 cc syringe attached into the anterior chamber and asperating the aqueous humor into the syringe. The site of the needle insertion was on the sclera, approximately one millimeter, or less, from the corneal-scleral junction. Approximately 100 grams of aqueous humor was removed.

After removal of the aqueous humor an approximate equal weight of the test solution was added to the same site from which the aqueous humor was drawn. A 26-gauge needle with a 1.0 cc syringe was used to inject the test and control materials. The amount of test material installed was sufficient to fill the anterior chamber until "normal" pressure was restored. The eyes of each animal were monitored for flare and cell response at 24, 48, and 72 hours post-injection. Biomicroscopic examination showed no flare or cell response to any of the test animals.

Example II: Vitreous Test on Cynomolgus Monkeys

Vitreous tests using the identical procedures of Example I were also performed on cynomolgus monkey eyes. The monkeys were also monitored at 24, 48 and 72 hour post-injection periods. Again, biomicroscopic examination showed no flare or cell response in any of the test animals.

Those skilled in the art will readily appreciate that the procedure of the present invention produces a high yield, sterile, non-inflammatory, heat resistant hyaluronic acid fraction of low molecular weight, which can be used in the same manner as previous hyaluronic acid fractions, without the need of non-inflammatory drugs and having the added advantage that it can be conveniently handled under aseptic conditions, such as high temperature, and subsequently cooled without a loss of its physical or chemical properties.

Although the present invention has been described in detail with reference to the presently-preferred embodiment, it should be understood by those of ordinary skill in the art that various modifications can be made without departing from the invention. Accordingly, the invention is limited only by the following claims.

**Claims**

1. A process for preparing a sterile, non-inflammatory, heat-resistant hyaluronic acid fraction which comprises precipitating hyaluronic acid from a crude source, denucleidizing and deproteinizing the precipitated hyaluronic acid, washing the denucleidized and deproteinized hyaluronic acid with a quaternaryalkyl or aryl amine by forming a hyaluronic acid-quaternaryalkyl or aryl amine complex, dissolving the hyaluronic acid-quaternaryalkyl or aryl amine complex with an excess of sodium dodecylsulfate and thereby releasing the hyaluronic acid into the sodium dodecylsulfate solution free of the quaternaryalkyl or aryl amine, and precipitating the freed hyaluronic acid to yield a sterile, pyrogen-free, protein-free, non-inflammatory, heat-resistant hyaluronic acid fraction.

2. A process according to Claim 1 wherein the quaternary-alkylamine is cetyl pyridinum chloride or 1-hexadecyltrimethyl ammonium bromide.

3. A process according to Claim 1 or 2 wherein the hyaluronic acid fraction has an average molecular weight of less than 750,000.

4. A process according to Claim 1, 2 or 3 wherein the hyaluronic acid is denucleidized and deproteinized by dissolving the precipitated hyaluronic acid in a sodium phosphate buffer at pH 7.2 or 7.4, heating the hyaluronic acid suspension to 35-38 degrees C, adding DNase and RNase and incubating until the nucleotides present in the solution are digested, and then added a 1% protease solution and incubating until the protease reaction is complete.

5. A process according to Claim 4 wherein the sodium phosphate buffer contains a magnesium compound and has a pH of 7.2.

6. A process according to any one of the preceding claims wherein hyaluronic acid is precipitated from a crude source with ethanol.

7. A process according to any one of the preceding claims wherein freed hyaluronic acid is precipitated from the sodium dodecylsulfate solution with ethanol.

8. A sterile, non-inflammatory, heat-resistant hyaluronic acid fraction having an average molecular weight of less than 750,000.

9. A hyaluronic acid fraction according to Claim 8 wherein the average molecular weight is at least 50,000.

10. The sodium salt of a hyaluronic acid fraction as claimed in Claim 8 or 9.

11. A composition comprising a hyaluronic acid fraction or salt as claimed in Claim 8, 9 or 10 dissolved in a physiologically acceptable buffer solution.

12. A composition according to Claim 11 wherein the buffer solution is PBS or physiological saline.

13. A composition according to Claim 11 or 12 wherein the hyaluronic acid concentration is 1.2 to 2.0 percent by weight.